Europäisches Patentamt

European Patent Office

Office européen des brevets

(19)

(11) **EP 1 117 482 B1**

(12) **EUROPÄISCHE PATENTSCHRIFT**

(45) Veröffentlichungstag und Bekanntmachung des Hinweises auf die Patenterteilung:
**10.04.2002 Patentblatt 2002/15**

(21) Anmeldenummer: **99946034.8**

(22) Anmeldetag: **26.08.1999**

(51) Int Cl.$^7$: **B01J 27/198**, C07D 307/60, C07C 51/215

(86) Internationale Anmeldenummer:
**PCT/EP99/06274**

(87) Internationale Veröffentlichungsnummer:
**WO 00/13793 (16.03.2000 Gazette 2000/11)**

(54) **VERFAHREN ZUR HERSTELLUNG VON KATALYSATOREN FÜR DIE SYNTHESE VON MALEINSÄUREANHYDRID DURCH GASPHASENOXIDATION**

METHOD FOR PRODUCING CATALYSTS FOR SYNTHESISING MALEIC ANHYDRIDE BY MEANS OF GAS PHASE OXIDATION

PROCEDE DE PRODUCTION DE CATALYSEURS DESTINES A LA SYNTHESE DE L'ANHYDRIDE DE L'ACIDE MALEIQUE PAR OXYGENATION EN PHASE GAZEUSE

(84) Benannte Vertragsstaaten:
**AT BE CH CY DE DK ES FI FR GB GR IE IT LI LU MC NL PT SE**

(30) Priorität: **03.09.1998 DE 19840224**

(43) Veröffentlichungstag der Anmeldung:
**25.07.2001 Patentblatt 2001/30**

(73) Patentinhaber: **Consortium für elektrochemische Industrie GmbH**
**81379 München (DE)**

(72) Erfinder:
• **GROKE, Dirk**
**D-82024 Taufkirchen (DE)**

• **BOSCH, Richard**
**D-82110 Germering (DE)**
• **LOTZ, Joachim**
**D-81369 München (DE)**
• **EBERLE, Hans-Jürgen**
**D-81477 München (DE)**

(74) Vertreter: **Rimböck, Karl-Heinz, Dr. et al**
**c/o Wacker-Chemie GmbH**
**Zentralabteilung PML**
**Hanns-Seidel-Platz 4**
**81737 München (DE)**

(56) Entgegenhaltungen:
EP-A- 0 036 623    GB-A- 2 118 060
US-A- 4 179 404

## Beschreibung

Die Erfindung betrifft ein Verfahren zur Herstellung von Katalysatoren für die partielle Gasphasenoxidation von gesättigten und/oder ungesättigten $C_4$-Kohlenwasserstoffen zu Maleinsäureanhydrid (MSA).

Die Herstellung von Maleinsäureanhydrid aus $C_4$-Kohlenwasserstoffen ist seit mehr als 20 Jahren bekannt. Dabei werden Katalysatoren auf Basis von Vanadylphosphaten (Vanadylpyrophosphat, Vanadium-Phosphor-Oxide) eingesetzt. Die Herstellung dieser Vanadylphosphate erfolgt über einen Katalysatorvorläufer (Precursor), der in wäßrigem oder organischem Medium hergestellt werden kann. Der Precursor wird dann in einem zweiten Schritt vor oder nach der Formgebung entweder im Reaktor (in-situ) oder extern in die eigentlich katalytisch aktive Substanz umgewandelt.

Die Umsetzung des $C_4$-Kohlenwasserstoffs am Katalysator erfolgt in verschiedenen Reaktortypen. Zum Einsatz kommen Festbett-, Wirbelschicht- und auch Riser-Reaktoren, wobei in Festbettreaktoren ausschließlich Katalysatoren auf der Basis von Vanadylphosphaten in Form von Vollkontakten eingesetzt werden. Aus EP-B 72381 und WO-A 96/25230 ist auch die Verwendung von Schalenkatalysatoren bekannt.

Die Herstellung der Precursor ist sowohl in wäßrigem sowie auch in organischem Medium möglich. In der Technik wird bevorzugt das Verfahren in organischem Lösungsmittel angewandt. Dabei hat sich iso-Butanol (2-Methyl-propan-1-ol) als Lösungsmittel bzw. Reaktionsmedium bewährt. Die in wäßrigem Medium hergestellten Katalysatoren weisen im Unterschied zu den in organischem Medium hergestellten deutlich geringere Aktivität und Selektivität auf. Eine Ursache für die Unterschiede liegt in der geringeren spezifischen Oberfläche der auf wäßrigem Wege hergestellten Katalysatoren. Die Verwendung eines organischen Lösungsmittel bringt weiterhin verfahrenstechnische Vorteile in Form einer leichteren Abtrennbarkeit des gebildeten Precursors von der Reaktionsmischung mit sich, da das Produkt im Lösungsmittel unlöslich ist. Das Lösungsmittel muß eine Reihe von Voraussetzungen erfüllen. So darf es zum Beispiel nicht mit den für die Bildung des Vanadylphosphates benötigten Phosphorverbindungen reagieren. Ferner muß sich sein Siedepunkt in einem Bereich bewegen, in dem die Bildung der gewünschten Vanadylphosphate beobachtet wird. Wie allgemein bekannt, beeinflußt die Reaktionstemperatur die Geschwindigkeit der Katalysatorbildung stark. Da die Reaktion meist im siedenden Lösungsmittel durchgeführt wird, spielt daher auch der Siedepunkt des Lösungsmittels eine wichtige Rolle.
Das Lösungsmittel besitzt ferner einen starken Einfluß auf die Morphologie des Reaktionsproduktes und teilweise sogar auf die sich bildenden Phasen (Doi, T.; Miyake, T.; Applied Catal. A. General 164 (1997), 141 - 148).

Als vanadiumhaltige Ausgangsverbindung wird in der Regel Vanadiumpentoxid eingesetzt. Als Phosphorverbindung dient in der Regel Phosphorsäure. Da das Vanadium in der Zielverbindung in der Oxidationsstufe IV vorliegt, ist eine Reduktion mit Hilfe eines geeigneten Reduktionsmittel notwendig. Hierfür werden eine Vielzahl von Verbindungen beschrieben. So kommen zum Beispiel gasförmiges HCl, Oxalsäure, Benzylalkohol, iso-Butanol, Hydrazin aber auch viele andere Reduktionsmittel zum Einsatz. Die Natur des Reduktionsmittels spielt dabei für die Reaktion eine untergeordnete Rolle.

US-A 4132670 beschreibt ein Verfahren zur Herstellung von Vanadylphosphat-Katalysatoren. Dabei wird Vanadiumpentoxyd in Alkohol, vorzugsweise Iso-Butanol zu einer Vanadin-(IV)-Verbindung umgesetzt. Die Reduktion von Vanadium (V) zu Vanadium (IV) geschieht dabei mittels des eingesetzten Lösungsmittels. Anschließend wird mit konzentrierter $H_3PO_4$ der Vanadiumphosphat Precursor hergestellt. Der Wassergehalt des Reaktionsgemisches bei der Herstellung des Precursors wird dabei als ein wichtiger Parameter beschrieben. Dabei wird ein niedriger Wassergehalt der Reaktionsmischung empfohlen.

Aus US-A 4382876 bzw. EP-A 0036623 ist ein Verfahren zu Herstellung von Katalysatoren bekannt, bei dem eine Vanadium-(V)-Verbindung in iso-Butanol als Lösungsmittel vorgelegt wird, und $H_3PO_3$ als Reduktionmittel in kleineren Mengen zugegeben wird. Die Reduktion der Vanadium-(V)-Verbindung zu Vanadium-(IV) geschieht dabei erfindungsgemäß aus einer kombinierten Reduktion aus Tertiärem Alkohol und Phosphoriger Säure. Das bei diesem Verfahren anfallende Wasser wird zusammen mit dem Lösungsmittel mehrmals diskontinuierlich abdestilliert. Aus dieser Verfahrensweise resultiert der Nachteil, daß sich im Reaktionsansatz immer wieder Mengen von Wasser bilden, die erst nach einer gewissen Zeit abdestilliert werden. Somit kann ein stetig geringer Wassergehalt nicht eingehalten werden, was zu einer schlechteren Performance des späteren Katalysators führt.

Aus WO-A 95/29006 ist ebenfalls ein Verfahren zur Herstellung von Vanadylphosphat Katalysatoren bekannt. Auch hier wird eine Vanadium-(V)-Verbindung mit Phosphorsäure in Isobutanol umgesetzt. Zur Steuerung des Wassergehalts wird in diesem Verfahren durch Zusatz von hochkonzentrierter Phosphorsäure zur Reaktionsmischung das gebildete Reaktionswasser abgefangen. Der gesamte Wassergehalt des Reaktionsgemischs wird dabei durch die verwendeten Phosphorverbindungen (85 % $H_3PO_4$, 100 % $H_3PO_4$, 106 % $H_3PO_4$ oder Polyphosphorsäuren) und durch das bei der Reaktion gebildete Wasser (durch die Reduktion von Vanadium(V)- zu Vanadium(IV)-Verbindungen) bestimmt. Ein großer Nachteil dieses Verfahrens sind die hohen Kosten und der schwierige Umgang mit konzentrierter Phosphorsäure und Polyphosphorsäure.

Die Umsetzung der Ausgangssubstanzen zum gewünschten Vanadylphosphat kann grundsätzlich auf verschiedenen Wegen erfolgen:

1. Reduktion der Vanadium(V)-Verbindung zum Vanadium(IV), anschließend Umsetzung mit der Phosphor-Verbindung zum Vanadyl(IV)phosphat,

2. Umsetzung der Vanadium(V)-Verbindung mit der Phosphorverbindung zum entsprechenden Vanadium(V)phosphat und anschließende Reduktion zum Vanadyl(IV)phosphat, oder

3. parallele Reduktion und Umsetzung mit der Phosphor-Verbindung zum Vanadyl(IV)phosphat.

Nach Abschluß der Umsetzung wird das in Suspension oder in Lösung vorliegende Vanadylphosphat durch geeignete Verfahren, wie beispielsweise Filtration oder Eindampfen, aus der Reaktionsmischung abgetrennt, getrocknet und in einem weiteren Schritt, der sogenannten Calcinierung, oder auch Aktivierung, in den aktiven Katalysator umgewandelt. Die Formgebung für den Einsatz in Festbettreaktoren erfolgt entweder vor oder nach der Calcinierung zum Beispiel durch Tablettierung oder durch Extrudieren. Eine weitere Möglichkeit zur Formgebung besteht in der Herstellung von Schalenkatalysatoren, wie sie auch bei anderen Prozessen wie beispielsweise der Synthese von Phthalsäureanhydrid aus o-Xylol oder Naphthalin zum Einsatz kommen.

Die Calcinierung des Precursors, d.h. die Überführung in den eigentlichen Katalysator kann sowohl extern als auch im Reaktor (in-situ), in dem die Umsetzung der $C_4$-Kohlenwasserstoffe zu Maleinsäureanhydrid durchgeführt wird, vorgenommen werden.

Aus US-A 4382876 ist ein Verfahren bekannt in dem der im ersten Schritt erhaltende Precursor in einem zweiten Schritt im Reaktor selbst in den eigentlichen Katalysator überführt wird. Dabei wird der Precursor zur Aktivierung mit einer Rampe von weniger als 1°C/min aufgeheizt um hot-spots im Katalysator zu vermeiden. Die in-situ Calcinierung erfolgt dabei unter Anwesenheit von $C_4$-Kohlenwasserstoff und Luft, wobei bis auf eine Temperatur von 450 °C bis 510 °C aufgeheizt wird. Nachdem diese Temperatur für ca. 12 bis 72 Stunden gehalten wurde, wird langsam wieder abgekühlt.

Ein großer Nachteil dieses Verfahrens ist, daß die entgültige Leistungsfähigkeit des Katalysators erst nach mehreren hundert Stunden erreicht ist.

Neben den beschriebenen Calcinierungsprozessen sind auch externe Formierungen bekannt. WO-A 95/29006 beschreibt ein Verfahren zur Calcinierung, in dem der Precursor in einer Atmosphäre aus Luft, Wasserdampf und Inertgas umgesetzt wird. Dabei wird der Precursor mit einer Aufheizrate von weniger als 1 °C/min in mehreren Schritten langsam auf eine Temperatur zwischen 350 °C und 550 °C aufgeheizt. Diese Temperatur wird für 2 bis 8 Stunden gehalten, wobei sich eine mittlere Oxidationsstufe des Vanadiums von kleiner + 4,5 ergibt. Aus USA 5185455 ist ein ähnliches Verfahren zur externen Calcinierung bekannt. Dabei wird der tablettierte Precursor unter definierter Gasatmosphäre stufenweise bis auf eine Temperatur von 425 °C aufgeheizt (erste Temperaturrampe bis 250 °C) und für einen Zeitraum von 6 Stunden auf dieser Temperatur belassen. Eine große Rolle spielt dabei der Sauerstoff- und Wassergehalt des Calciniergases im Hinblick auf die spätere Leistungsfähigkeit des Katalysators.

Diese Verfahren haben zum einen ökonomische Vorteile, da sie den Reaktor nicht wie bei der in-situ Calcinierung über mehrere Wochen, bis zur vollständigen Formierung des Katalysators, mit geringerer Ausbeute blockieren. Zum anderen kann bei der externen Calcinierung der Precursor in kleineren Teilmengen formiert werden, was eine laufende Qualitätskontrolle erlaubt und dadurch nicht die gesamte Katalysator Charge dem Risiko eines Verlustes ausgesetzt ist. Durch die externe Calcinierung lassen sich die für eine optimale Formierung des Precursors zum aktiven Katalysator benötigten Bedingungen (z.B. Sauerstoffgehalt und Temperatur) einheitlicher und besser sicherstellen. Demgegenüber führt beispielsweise die in-situ Calcinierung im Festbettreaktor, zu einer über den Reaktor bzw. die Reaktorrohrlänge uneinheitlichen Umwandlung des Katalysators.

Allgemein wird bei der externen Calcinierung der Precursor zumeist unter definierten Gasatmosphären (Gasmischungen aus Sauerstoff, Inertgas und Wasserdampf) und einem genau festgelegten Temperaturprogramm umgesetzt. Der sich bei diesem Vorgehen bildende Katalysator weist im Unterschied zu den in-situ calcinierten Kontakten sofort seine volle Leistungsfähigkeit auf. Der Sauerstoffgehalt (Steuerung der mittleren Oxidationsstufe des Vanadiums), die Konzentration von Wasser in der Ofenatmosphäre und die Parameter des Temperaturprogramms (Steilheit der Temperaturrampen, Endtemperaturen und Haltezeiten) sind wichtige Einflußgrößen, die die Leistungsfähigkeit der erhaltenen Katalysatoren beeinflussen.

Zur Steuerung der katalytischen Eigenschaften der Katalysatoren, werden in US-A 4132670, EP-A 0458541, DE-A 3018849 (US-A 4251390), WO-A 93/01155, US-A 5288880 der Zusatz vieler Verbindungen als Promotoren oder Dotierstoffe in stark unterschiedlichen Konzentrationen vorgeschlagen. Zum Einsatz kommen dabei beispielsweise Verbindungen der Elemente Mo, Zn, Fe, Co, Li, Ce, Zr, U, Bi und Cr wobei Atomzahlverhältnisse Vanadium : Promotorelement im Bereich von 1 : 0,2 bis 0,001 beschrieben werden. Der Promotorzusatz kann dabei zu verschiedenen Zeitpunkten des Produktionsverfahrens erfolgen. Angewendet werden Techniken bei denen der Zusatz während der Umsetzung zum Precursor erfolgt (homogene Verteilung des Promotors im Precursor) oder auch Imprägniermethoden, mit denen der zuvor synthetisierte Precursor oberflächlich mit dem Promotor versehen wird.

Zur Steuerung der Struktur des sich bildenden Katalysators wird in EP-A 0151912 der Einsatz von oberflächenaktiven, alkoholmodifizierenden Substanzen, wie beispielsweise HI, $SO_2$ oder rauchende Schwefelsäure beschrieben.

Neben dem Einsatz von Promotoren wird auch die Beimischung von inerten Materialien zur Steuerung der Katalysatoraktivität beschrieben. Als inerte Zusatzstoffe werden beispielsweise $SiO_2$, $TiO_2$ und Siliziumcarbid aufgeführt. Die Zugabe dieser Stoffe erfolgt in der Regel nach beendeter Synthese des Precursors und vor der Formgebung des Katalysators.

Ferner wird in neuerer Zeit auch die Verwendung von Zusätzen, die die Porenstruktur des eingesetzten Katalysator-Formkörpers steuern, beschrieben. Hierbei werden dem getrockneten Precursor vor der Formgebung beispielsweise gewisse Mengen an höheren Alkansäuren zugesetzt. In einem zusätzlichen Prozeßschritt werden diese Substanzen nach der Formgebung schließlich wieder aus dem Formkörper entfernt, wodurch eine definierte Porenstruktur erreicht werden kann.

Die beschriebenen Verfahren zur Herstellung von Katalysatoren für die Synthese von Maleinsäureanhydrid weisen alle eine Reihe von Nachteilen auf. So besteht beispielsweise durch die Reduktion von Vanadium V mittels Chlorwasserstoff-Gas eine erhöhte Korrosion innerhalb der Anlagen. Die entstehenden Chlorabgase und Chloridabfälle müssen teuer entsorgt werden und verunreinigen zum Teil den Precursor. Ein weiterer Nachteil ist mit den reaktionsbedingt entstehenden Mengen an Wasser verbunden. Die im Stand der Technik beschriebenen Verfahren zur Entfernung von Wasser aus dem Reaktionsgemisch verschlechtern entweder die Performance des späteren Katalysators, oder sind nur mit hohen Kosten und Aufwand, wie beispielsweise mit Polyphosphorsäure, durchführbar.

Es bestand daher die Aufgabe ein Verfahren zur Herstellung von Katalysatoren für die Synthese von Maleinsäureanhydrid durch Gasphasenoxidation zu entwickeln, daß die genannten Nachteile ausschließt.

Gegenstand der Erfindung ist ein Verfahren zur Herstellung von Katalysatoren für die Synthese von Maleinsäureanhydrid durch Oxidation von gesättigten und/oder ungesättigten $C_4$-Kohlenwasserstofen auf der Basis von Vanadylphosphaten durch Umsetzung einer Vanadium(V)-Verbindung mit einer Mischung aus Phosphoriger- und Phosphorsäure in einem Lösungsmittelgemisch, dadurch gekennzeichnet, daß das molare $H_3PO_3/H_3PO_4$-Verhältnis 1:1 bis 1:2,5 ist, als Lösungsmittel ein Gemisch aus Iso-Butanol, einem Strukturbildner aus der Gruppe enthaltend, ein- und mehrfunktionelle Alkohole, ein- und mehrfunktionelle Amine, organische Phosphate, Phosphite und Phosphonate, einem Schleppmittel aus der Gruppe enthaltend, Alkylaromaten und Cycloalkane, wobei das Molverhältnis zwischen Vanadium und Strukturbildner in einem Bereich zwischen 10 : 1 und 100 : 1 liegt, eingesetzt wird und bei Siedetemperatur das Reaktionswasser mit Schleppmittel kontinuierlich ausgekreist wird, wobei der Restwassergehalt des Rücklaufs und der sich daraus ergebende Restwassergehalt in der Reaktionsmischung unter 0,5 Vol.-% liegt und der so erhaltene Precursor anschließend einer Calcinierung unterzogen wird, bei der ein Temperaturprogramm bestehend aus 3 Rampen zum Einsatz kommt, wobei die Rampen wie folgt gestaltet werden:

→ Rampe 1: Starttemperatur bei ≤ 50°C, Aufheizrate 5 bis 20°C/min, Endtemperatur 100 bis 250°C, Haltezeit 0 bis 3 h,

→ Rampe 2: Starttemperatur bei Endtemperatur Rampe 1, Aufheizrate 1 bis 10°C/min, Endtemperatur 150 bis 300°C, Haltezeit 0 bis 3 h,

→ Rampe 3: Starttemperatur bei Endtemperatur Rampe 2, Aufheizrate 0,1 bis 3°C/min, Endtemperatur: 380 bis 460°C, Haltezeit 2 bis 8 h,

und während dieser Temperaturrampen Gasgemische aus Luft, Inertgas und Wasserdampf in einem Volumen-Mischungsverhältnis zwischen 0,1 bis 0,5 : 0,1 bis 0,5 : 0,0 bis 0,8 eingesetzt werden.

Eine Vanadium-(V)-Verbindung wird in einer Mischung aus einem die Struktur des Vanadylphosphat beeinflussenden Agens, beispielsweise Benzylalkohol, iso-Butanol und einem Hilfsstoff, der die Entfernung des gebildeten und / oder eingetragenen Wassers ermöglicht (sogenanntes "Schleppmittel") suspendiert. Die Suspension wird aufgeheizt und nach Erreichen des Siedepunktes wird eine Mischung aus Phosphoriger Säure und Phosphorsäure zugegeben. Als Vanadium-(V)-Verbindung wird bevorzugt Vanadiumpentoxid eingesetzt. Auch andere Vanadium-(V)-Verbindungen wie beispielsweise deren Oxychloride können verwendet werden.

Als Phosphorkomponenten werden Phosphorige Säure und Phosphorsäure benutzt. Die Phosphorige Säure dient sowohl als Reduktionsmittel für das Vanadium(V) als auch als Phosphorquelle. Der Einsatz eines weiteren Reduktionsmittels entfällt dabei. Die Phosphorige Säure wird bevorzugt in fester Form zur Erstellung der Mischung eingesetzt, kann aber auch als Lösung verwendet werden. Bevorzugt wird eine Phosphorsäure mit 85 bis 100 Gew.-%, besonders bevorzugt mit 85 Gew.-%. Die Phosphorsäure-Mischung wird durch Auflösen der Phosphorigen Säure in der Phosphorsäure, gegebenenfalls unter leichtem Erwärmen, hergestellt. Die Mischung aus $H_3PO_3/H_3PO_4$ besitzt ein molares Mischungsverhältnis von 1 : 1 bis 1 : 2,5, bevorzugt 1 : 1,5 bis 1 : 2,0.

Das Atomzahlverhältnis Vanadium : Phosphor zwischen der eingesetzten Vanadiummenge und der Phosphormenge liegt im Bereich von 1 : 0,9 bis 1 : 2,0. Die so erhaltene Vanadium-Phosphor-Verbindung besitzt eine mittlere Vanadiumoxidationsstufe von 3,85 bis 4,3, wobei das molare Vanadiumoxidationsstufe von 3,85 bis 4,3, wobei das molare Vanadium-Phosphor-Verhältnis in der isolierten Verbindung im Bereich zwischen 1 : 0,9 und 1 : 1,1 liegt.

Die Zugabe der Mischung aus phosphoriger Säure und Phosphorsäure erfolgt direkt nach Erreichen des Siedepunktes der Vanadiumpentoxid/Lösungsmittel-Suspension. Eine "Vorreduktionsperiode" wie sie in anderen Patenten beschrieben wird, wird nicht angewandt.

Nach Abschluß der Zugabe der Phosphorkomponenten wird der Reaktionsansatz für einen Zeitraum von 4 h bis 24 h, bevorzugt zwischen 12 h und 20 h, unter Rückflußbedingungen gehalten.

Als Lösungsmittel in dem die Reaktion durchgeführt wird kommt iso-Butanol (2-Methyl-propan-1-ol) zum Einsatz. Angewendet können aber auch andere inerte Lösungsmittel mit einem Siedepunkt von ca. 100°C, beispielsweise Toluol. Ein entscheidender Punkt des beanspruchten Verfahrens besteht darin, daß der Zusatz eines strukturmodifizierenden Agens nur in geringen Mengen notwendig ist.

Als Strukturmodifizierer können folgende Verbindungsklassen einsetzt werden: ein- und mehrfunktionelle Alkohole, beispielsweise Ethandiol, Propan-1,3-diol, n-Hexanol, Diphenylmethanol, bevorzugt Benzylalkohol und alpha-omega-Alkandiole, ein- und mehrfunktionelle Amine, beispielsweise n-Propylamin, iso-Butylamin, Ethylendiamin, bevorzugt n-Decylamin und Propylendiamin, organische Phosphate, beispielsweise Butylphosphat, Dibutylphosphat, Trimethylphosphat, bevorzugt Tributylphosphat, Phosphite oder Phosphonate, beispielsweise Methylphosphonsäure, Trimethylphosphit, Dibutylphosphonsäure, bevorzugt Tributylphosphit, oder auch deren Mischungen. Diese Verbindungen können aufgrund ihrer chemischen Struktur in die Schichtstruktur des Precursors eingebaut (intercaliert) werden und so die Morphologie des Materials, d. h. die Art und die Anzahl der gebildeten Fehlstellen beeinflussen. Bezogen auf die Molzahl beträgt das Verhältnis des Strukturmodifizierers zum eingesetzten Vanadium 0,1 % bis 10 %. Die geringe benötigte Menge unterscheidet das hier beschriebene Verfahren deutlich von anderen Herstellungsmethoden bei denen die Zusätze, insbesondere der Zusatz von Benzylalkohol, im molaren Verhältnis Vanadium : Zusatz bei 1 : 0,5 bis 1: 1,0 liegen. Diese zugesetzten Substanzen dienen dort als Reduktionsmittel, wobei ein strukturmodifizierender Einfluß bei Einsatz geringer Mengen nicht beschrieben wird (EP-A 0151912).

Dem Reaktionsgemisch werden weiterhin sogenannte "Schleppmittel" zugesetzt, die es ermöglichen, daß während der Reaktion gebildetes Wasser aus der Reaktionsmischung entfernt werden kann. Als Schleppmittel können dabei eine Reihe von Substanzen zum Einsatz kommen. Im allgemeinen werden dazu unpolare Verbindungen, die mit Wasser nur in geringem Umfang mischbar sind eingesetzt, beispielsweise Alkylaromaten und Cycloalkane. Bevorzugt werden aufgrund der einfachen Verfügbarkeit Toluol, Xylole, Cyclohexan und Benzol eingesetzt. Besonders bevorzugt ist Cyclohexan als Schleppmittel. Die dem iso-Butanol zugesetzte Menge bewegt sich in einem Bereich von 5 bis 15 Vol.-%, bevorzugt 8 bis 12 Vol.-% der eingesetzten Lösungsmittelmenge.

Während des Siedens der Reaktionsmischung wird das ternäre Gemisch aus Lösungsmittel, Schleppmittel und entstandenem Reaktionswasser kontinuierlich über einen externen Kreislauf aus dem Reaktor geführt. Innerhalb dieses Kreislaufs wird das ternäre Azeotrop in einen Behälter geleitet, der die Trennung des 2-phasigen Kondensats und damit die Abtrennung der wasserreichen Phase gestattet. Das an Wasser verarmte Gemisch aus Lösungsmittel und Schleppmittel wird dem Reaktor wieder zugeführt. Nach der Abtrennung beträgt der Restwassergehalt des zurückgeführten Gemisches weniger als 0,5 Vol.-%. Durch dieses Verfahren ist gewährleistet, daß der Wassergehalt der Reaktionsmischung stetig unter 0,5 Vol.-% gehalten wird. Dies ist ein entscheidendes Kriterium in Bezug auf die Selektivität und Umsatz des fertigen Katalysators.

Bei der erfindungsgemäßen Herstellung des Precursors kann gegebenenfalls auch eine Dotierung des Katalysators erfolgen. Eine Dotierung des Katalysators kann auf verschiedenen Wegen erfolgen. Zum einen können dem organischen Lösungsmittel-Gemisch Metallverbindungen in Form löslicher Verbindungen der Elemente Mo, Wo, Bi, Cr, Co, Ni, Fe, Li, Ce, Zr, U und Zn, bevorzugt Mo, Cr, Bi, Co, Zn, Li und Ce, in einem Atomzahlverhältnis Vanadium : Dotierelement von 1 : 0,1 bis 0,001 zugesetzt werden. Ein weiterer Weg besteht in der Dotierung des gebildeten Katalysatorvorläufers nach Abschluß der Reaktion unmittelbar vor der Trocknung bzw. der Abtrennung. Auch hier müssen, um eine gleichmäßige Verteilung des Dotierstoffs zu erreichen, lösliche Verbindungen eingesetzt werden. Beispiele für lösliche Verbindungen sind die Chloride, Carbonate, Acetylacetonate, Acetate und Nitrate der obengenannten Metalle.

Nach Ablauf der Rückflußzeit erfolgt die Abtrennung des Precursors. Dies kann über Filtration und anschließende Trocknung erfolgen. Besonders vorteilhaft kann dies dabei durch das Verfahren der Sprühtrocknung der gewonnenen Suspension geschehen.

Nach der Trocknung des Precursors kann dieser direkt zur weiteren Verarbeitung zu Schalenkatalysatoren, zu Katalysatoren für Prozesse in fluidisierten Katalysatorschüttungen oder zu Vollkontaktenkatalysatoren für Festbettreaktoren eingesetzt werden. Dazu ist es jedoch notwendig den Precursor in eine für das jeweilige Anwendungsgebiet vorteilhafte Form zu bringen. Diese Formgebung kann zum Beispiel durch Tablettierung, Agglomeration, Extrusion oder einen ähnlichen Prozeß erfolgen. Die Natur des Formgebungsprozesses und die daraus resultierende Katalysatorform und Beschaffenheit hat keinen Einfluß auf die Anwendbarkeit des Verfahrens. Zur Formgebung können Hilfsstoffe, die beispielsweise die Formbarkeit und Rieselfähigkeit des Rohmaterials beeinflussen, zugesetzt werden. Als Hilfsstoffe dienen beispielsweise Graphit, höhere Alkansäuren (z.B. Stearinsäure), Polyethylenglykole und Kieselsäuren, oder deren Mischungen. Als Form der Katalysatorkörper kommen beispielsweise Zylinder, Kugel und Ringe in Betracht.

Im Anschluß wird der Precursor unabhängig von seiner erhaltenen Form einem Calcinierprozess unterzogen, wobei er in die eigentlich katalytisch aktive Form umgewandelt wird. Die Calcinierung kann dabei sowohl im Reaktor (in-situ) als auch in einem externen Schritt erfolgen. Die externe Calcinierung ist dabei zu bevorzugen, da diese zu Katalysatoren führt, die direkt im Anschluß an das Verfahren voll einsatzfähig sind. Eine längere Formierungsphase, wie sie bei in-situ calcinierten Katalysatoren beobachtet wird, entfällt dabei.

Die im Rahmen des hier geschilderten Verfahrens durchgeführte Calcinierung wird mit dem getrockneten Precursor als Pulver oder Formkörper bevorzugt in folgender Weise durchgeführt:

In einem geeigneten Ofensystem, beispielsweise einem Wirbelschichtofen bei Pulvern, oder einem Horden- oder Röhrenofen bei Formkörpern, das mit einer Einheit zur Dosierung von Gasen und zur zeitlichen Temperatursteuerung ausgerüstet ist, wird der Precursor eingefüllt. Die im folgenden beschriebenen Parameter beziehen sich dabei auf die externe Calcinierung von Vollkontakten:

**1. Calcinierschritt: Inertisierung**

In einem ersten Schritt erfolgt die Inertisierung des Ofenraums bei einer Temperatur im Bereich zwischen 20°C und 100°C unter Einsatz eines Inertgases. Als Inertgas wird bevorzugt Stickstoff eingesetzt. Die für die Inertisierung benötigte Zeit und Menge des Inertgases sind so zu bemessen, das ein Restgehalt von Sauerstoff im Ofensystem von weniger als 5 Vol.-% gewährleistet wird.

**2. Calcinierschritt: Rampe 1**

Im nächsten Schritt wird das Ofensystem mit einer Gasmischung, die einem Sauerstoffgehalt von 5 Vol.-% bis 20 Vol.-% besitzt, beaufschlagt. Die Ofentemperatur wird nun mit einer Geschwindigkeit von 5°C/min bis 20°C/min (Rampe 1) bis zu einer Temperatur von 150°C bis 250°C (Endtemperatur 1) aufgeheizt. Gegebenenfalls kann diese Temperatur für einen Zeitraum von bis zu 3 Stunden konstant gehalten werden.

**3. Calcinierschritt: Rampe 2**

Nach Erreichen der Endtemperatur 1 und Ablauf der Haltezeit wird das Aufheizen des Systems mit einer Rampe 2 bis zu einer Endtemperatur 2 von 200°C bis 300°C fortgesetzt. Die Aufheizgeschwindigkeit liegt dabei zwischen 1°C/min und 10°C/min und das dem Ofensystem zugeführte Gasgemisch besteht aus den Komponenten Sauerstoff, Wasserdampf und Inertgas, wobei ein Sauerstoffgehalt von 5 Vol.-% und ein Wassergehalt von 50 Vol.-% erreicht wird. Nach Erreichen der Endtemperatur kann auch bei diesem Schritt eine Haltezeit von bis zu 3 Stunden eingelegt werden.

**4. Calcinierschritt: Rampe 3**

Nach Erreichen der Endtemperatur 2 und Ablauf der Haltezeit wird das Aufheizen des Systems mit einer Rampe 3 bis zu einer Endtemperatur 3 von 380°C bis 460°C fortgesetzt. Dabei wird ein Gasgemisch entsprechend der Zusammensetzung aus Rampe 2 eingesetzt. Die Aufheizgeschwindigkeit liegt dabei zwischen 0,1°C/min und 3°C/min. Diese Endtemperatur wird für einen Zeitraum von 2 h bis 8 h unter Beibehaltung der Gaszusammensetzung gehalten.

**5. Calcinierschritt: Abkühlphase**

Nach Abschluß des 4. Calcinierschrittes wird das Ofensystem unter Inertgas auf Umgebungstemperatur abgekühlt. Nach Unterschreiten einer Temperatur von 100°C kann das Ofensystem geöffnet und der Katalysator entnommen werden. Der Katalysator wird in gut schließende Gefäße abgefüllt.

Die Zugabe von Wasserdampf während der Calcinierung ist von großer Bedeutung, da sie die Umwandlung des Precursors in das katalytisch aktive Vanadylpyrophosphat positiv beeinflußt. Ohne Wasserdampf behandelte Proben weisen eine deutlich geringere katalytische Leistung auf. Als sauerstoffhaltiges Gas wird Luft bevorzugt. Die für die einzelnen Calcinerschritte benötigten Sauerstoffkonzentrationen können bevorzugt durch Mischung von Luft mit einem Inertgas, bevorzugt Stickstoff, erzielt werden. Das Gasgemisch aus Luft, Inertgas und Wasserdampf kann dabei in einem Volumen-Mischungsverhältnis zwischen 0,1 bis 0,5 : 0,1 bis 0,5 : 0,0 bis 0,8 eingesetzt werden.

Im Vergleich zum Stand der Technik unterscheidet sich die hier angewandte Calciniermethode deutlich hinsichtlich der verwendeten Temperaturrampen. Zur zeitlichen Verkürzung des Calcinierprozesses erfolgt bis zu einer Temperatur von 150°C bis 250°C die Aufheizung des Ofensystems mit einer gegenüber den Vergleichsschriften deutlich erhöhten Temperaturrampe. In der vorliegenden Anmeldung wird eine Temperaturrampe von 1 bis 10°C /min verwendet. Durch diese bis zu 10-fache Aufheizgeschwindigkeit degenüber dem Stand der Technik konnte ein Calcinierverfahren bereitgestellt werden, das durch einen Zeitgewinn gegenüber vergleichbaren Verfahren bringt und dadurch ökonomischer arbeitet. Unterschiede sind darüber hinaus hinsichtlich der Gaszusammensetzung in den einzelnen Calcinierphasen vorhanden.

Im folgenden soll das Verfahren zur Herstellung von MSA-Katalysatoren anhand von Beispielen erläutert werden:

**Beispiel 1: Erfindungsgemäße Herstellung von MSA-Katalysatoren**

In eine Mischung aus 2500 ml iso-Butanol, 250 ml Cyclohexan und 16,2 g Benzylalkohol (als strukturmodifizierendes Agens) wurde 272,8 g Vanadiumpentoxid unter Rühren in einem 4 1 Kolben mit Rührer, Thermometer, Tropftrichter und Wasserabscheider eingetragen. Nach Aufheizen des Ansatzes auf Siedetemperatur wurde eine Lösung von 90,4 g Phosphoriger Säure in 225,7 g 85%iger Phosphorsäure über den Tropftrichter innerhalb von 2 h zugegeben. Gleichzeitig mit der Zugabe der Phosphorsäure-Mischung wurde über den Wasserabscheider das mit der Säuremischung eingetragene bzw. das über die Reaktion gebildete Wasser entfernt. Die Rückflußbedingungen wurden bei gleichzeitigem Auskreisen von Wasser über einen Zeitraum von 16 h gehalten. Während dieser Zeit ändert sich die Farbe der Reaktionsmischung langsam von orange über grün bis zu einem leuchtenden blau. Innerhalb der Rückflußzeit wurden über den Wasserabscheider 97 g wäßrige Phase abgetrennt. Nach Ablauf der 16 h wurde der Wasserabscheider durch einen Kühler ersetzt und insgesamt ca. 1700 ml Lösungsmittel abdestilliert. Die Trocknung des so erhaltenen Produkts (Precursor) wurde bei 100 bis 150°C im Vakuumtrockenschrank vorgenommen. Man erhielt ca. 550 g Precursor.

Das Precursormaterial wurde zu Formkörpern weiter verarbeitet. Dazu wurden 250 g getrocknetes Precursor-Pulver mit 10 g Graphit versetzt und intensiv durchmischt. Der zugesetzte Graphit muß gleichmäßig in der Masse verteilt sein. Nach der Durchmischung wurden mit Hilfe einer Tablettiermaschine (Typ: Fette Exakta E1) zylindrische Presslinge mit einem Durchmesser von 5 mm und einer Höhe von 5 mm gefertigt. Das Gewicht der Presslinge lag bei ca. 120 mg.

Die Calcinierung der Formkörper wurde nach dem in Tabelle 1 aufgefürten Temperatur- und Gasmengenprogramm vorgenommen:

Tabelle 1:

| Calcinierprogramm Beispiel 1 | | | | | |
|---|---|---|---|---|---|
| Schritt | Rampe °C/min | Haltezeit min | Endtemperatur °C | Gaszusammensetzung Luft:$N_2$:$H_2O$ | Gesamtgasmenge l/h |
| 1. Inertisierung | - | 30 | 20 | -:1:- | 400 |
| 2. Rampe 1 | 10 | - | 150 | 1:1:- | 400 |
| 3. Rampe 2 | 5 | - | 250 | 0,5:0,5:1 | 400 |
| 4. Rampe 3 | 0,5 | 240 | 420 | 0,5:0,5:1 | 400 |
| 5. Abkühlen | Freies Abkühlen keine aktive Kühlung | - | 20 | -:1:- | 400 |

**Bestimmung der katalytischen Eigenschaften von MSA-Katalysatoren:**

In einem elektrisch beheizten Röhrenofen wurde in einem Quarzrohr (Innendurchmesser: 19 mm) eine 11 cm lange Schüttung des zu untersuchenden Katalysators hergestellt (Schüttungsvolumen: 31,2 ml). Die Einwaage am Katalysator wurde registriert. Zusätzlich wurde ein Thermoelement (Ni/CrNi) in der Schüttung plaziert, um eine Messung der Reaktionstemperatur zu ermöglichen. Zur Dosierung der Gase Luft und Butan wurden ein Rotameter bzw. ein Massenfluß-Regler (Fa. Brooks, Typ: 5850E) benutzt. Für die Versuche wurde eine Luftmenge von 600 $ml_n$/min und 5 $ml_n$/min n-Butan entsprechend einer Butan-Konzentration von 0,83 Vol.-% und einer Gasgeschwindigkeit von

1165 h$^{-1}$ verwendet. Das gebildete Maleinsäureanhydrid wurde in einer mit Wasser gefüllten Waschflasche über einen bekannten Zeitraum aufgefangen und durch Titration gegen 0,1 n NaOH mit Phenolphthalein als Indikator tritrimetrisch bestimmt. Durch eine entsprechende Ventilschaltung konnte sowohl das Eingangsgasgemisch als auch das Abgas, nach Entfernung der kondensierbaren Anteile, mit Hilfe eines Flammenionisationsdetektors (= FID, umgebauter Gaschromatograph Hewlett-Packard HP 5890 II) analysiert und damit der Umsatz bestimmt werden. Aus der gebildeten MSA-Menge pro Zeit und der in dieser Zeit eingesetzten Butanmenge wurden Ausbeute, Umsatz und Selektivität berechnet.

Umsatz, Ausbeute und Selektivität ergeben sich nach folgenden Beziehungen:

Umsatz:

$$U[\%] = \frac{Int(ein,C_4) - Int(aus,C_4)}{Int(ein,C_4)}$$

Ausbeute:

$$A[mol\%] = \frac{n(aus,MSA)}{n(ein,C_4)}$$

$$= \frac{Verbrauch(0,1n\ NaOH\ /\ ml)\ /\ 2}{v(ein,C4) * t}$$

Selektivität:

$$S[\%]=A/U$$

Da die Volumenänderung bei der Reaktion sich im Bereich von wenigen Prozent bewegt und neben CO und $CO_2$ Nebenprodukte nur in sehr geringen Mengen gebildet wurden, konnte die Ausbeuteberechnung gemäß obiger Gleichung durchgeführt werden.

**Erläuterung der verwendeten Symbole:**

$U$ : Umsatz
$A$ : Ausbeute
$S$ : Selektivität
Int(ein/aus, X) : Intensität FID-Signal Ein- bzw. Ausgang n(ein/aus, X) : Molzahl der Komponente X am Ein- bzw. Ausgang (mol)
v(ein,$C_4$): Gasfluß n-Butan in mol/h = c(ein,$C_4$)*v(ein,gesamt) c(ein,$C_4$) = 0,83 Vol.-%, v(ein,gesamt) = 27 mmol/h= 605 ml$_n$/h
t: Reaktionszeit in h

Eventuell gebildete Nebenprodukte und deren Konzentration ließen sich durch Analyse des Waschwassers mit Hilfe der Ionenchromatographie bestimmen. Während der Testläufe wurde darauf geachtet, daß die Katalysatortemperatur 480°C nicht überschreitet, da ansonsten eine Schädigung des Katalysators zu befürchten war. Zur Beurteilung der Katalysatoren wurde die Temperatur des Ofens solange variiert, bis die maximale Ausbeute erreicht wurde. Die Temperatur und der Umsatz bei maximaler Ausbeute wurden zur Beurteilung der Katalysatoren herangezogen.

**Beispiel 2 (Vergleichsbeispiel): Herstellung eines MSA-Katalysator gemäß WO-A 95/29006**

In einem 10 l Vierhalskolben mit Rührer, Thermometer, Rückflußkühler und Heizung wurde eine Mischung aus 6480 ml (= 5196 g) iso-Butanol, 720 ml (= 750 g) Benzylalkohol gegeben. Die Mischung wurde unter Rühren mit 670 g Vanadiumpentoxid versetzt. Die Reaktionsmischung wurde nun bis zum Rückfluß erhitzt und für 3 Stunden unter diesen Bedingungen gehalten. Anschließend ließ man die Mischung auf ca. 20°C unter der Siedetemperatur abkühlen und gab dann 816 g 106%ige frisch hergestellte Phosphorsäure hinzu. Die so erhaltende Mischung wurde nun erneut bis zum Rückfluß erhitzt und für 16 Stunden unter diesen Bedingungen gehalten. Nach Abschluß der 16 Stunden wurde die Reaktionsmischung auf ca. 50°C abgekühlt und filtriert. Man erhielt einen leuchtend-blauen Filterkuchen, der in Schalen umgefüllt und bei 150°C für 10 Stunden in einem Umluftofen getrocknet wurde. Man erhielt ca. 1300 g eines graublauen Katalysatorprecursor-Pulvers.

Das getrocknete Pulver wurde unter leichtem Druck durch ein 65-mesh Sieb gegeben, mit ca. 4 Gew.-% Graphit versetzt und in zylindrische 4*4 mm große Tabletten mit Hilfe einer Tablettiermaschine (Typ: Fette Exakta E1) gepreßt. Die so erhaltenen Presslinge wurden gemäß folgenden Bedingungen calciniert.

100 ml Precursor-Tabletten wurden in einem Glasrohr mit 5 cm Durchmesser in einen Röhrenofen gegeben. Vor Beginn des Temperaturprogrammes wurde ein Gasgemisch (160 l/h) mit einem Sauerstoffgehalt von 5 Vol.-% als Luft/Stickstoffmischung durch den Ofen geleitet. Das Ofensystem wurde nun mit einer Heizrate von 0,5°C/min bis zu einer Temperatur von 150°C aufgeheizt. Nach Erreichen von 150°C wurde die Zusammensetzung der Gasmischung geändert, und zwar der Gestalt, daß ein Gasgemisch mit einer Zusammensetzung von 25 Vol.-% Luft, 25 Vol.-% Stickstoff und 50 Vol.-% Wasserdampf eingesetzt wurde. Die Temperatur wurde nun weiter bis auf 420°C erhöht und für einen Zeitraum von 4 Stunden gehalten. Nach dem Abkühlen wurde der Katalysator wie unter Beispiel 1 aufgeführt auf seine katalytische Leistung getestet.

**Beispiel 3, (Vergleichsbeispiel): Herstellung eines MSA-Katalysators gemäß EP-A 0036623 (US-A 4382876)**

In 2,8 l iso-Butanol wurden 103,3 g Phosphorige Säure und 352,8 g 85%ige Phosphorsäure gelöst. In der erhaltenen Lösung wurden 327,4 g Vanadiumpentoxid aufgeschlämmt und der Reaktionsansatz auf Siedetemperatur erhitzt. Vom Rückfluß wurden innerhalb von 30 bis 60 min ca. 500 ml Kondensat entnommen. Im Anschluß verblieb der Ansatz weitere 5 h unter Rückflußbedingungen. Nach Abkühlen auf Umgebungstemperatur wurde filtriert und das hellblaue Reaktionsprodukt für 12 h bei 130°C im Vakuum (25 bis 50 hPa) getrocknet. Man erhielt etwa 680 g eines grauen Katalysatorvorläufer-Pulvers. Dieses Pulver wurde nun mit 3 Gew.-% Graphit versetzt und zu Presslingen mit 5 mm Durchmesser und 5 mm Höhe verpreßt.

Die Calcinierung des Precursors wurde bei diesem Verfahren in-situ d.h. im Pilot-Reaktor in folgender Weise durchgeführt: Das Reaktionsrohr wurde mit den Katalysator-Precursor-Tabletten gefüllt (Füllhöhe: 240 cm, Innendurchmesser: 25 mm). Anschließend wurde der Katalysator mit 500 l/h Luft durchströmt und der Reaktor auf 200°C aufgeheizt. Unter weiterem Durchleiten von Luft wurde die Temperatur des Reaktors innerhalb von 26 h von 200°C auf 330°C erhöht. Bei Erreichen der 330°C wurde ein Gasgemisch bestehend aus $C_4$-Kohlenwasserstoffen (ca. 70 Vol.-% Butene, 30 Vol.-% n-Butan) mit einer Konzentration von 1,45 Vol.-% bei einer Gasgeschwindigkeit von 1700 h$^{-1}$ über den Katalysator geleitet. Die Ofentemperatur wurde nun soweit erhöht, bis eine Hot-spot-Temperatur von 500°C erreicht wurde. Nun wurde das $C_4$-Luft-Gemisch durch ein Butan-Luft-Gemisch gleicher Konzentration ersetzt. Beim Wechsel zum Butan-Luft-Gemisch sank die Hot-spot-Temperatur ab, so daß eine erneute Anpassung der Ofentemperatur notwendig war, bis wiederum eine Hot-spot-Temperatur von 500°C erreicht wurde. Diese Temperatur wurde über einen Zeitraum von 24 h gehalten und die Gasgeschwindigkeit von 1700 h$^{-1}$ auf 2300 h$^{-1}$ unter Beibehaltung des Butan-Luftgemisches erhöht. Innerhalb von 8 bis 14 Tagen fand eine weitere Aktivierung des Katalysators statt, wobei darauf geachtet werden mußte, daß der Umsatz des Butans 90 % nicht überschritt. Der Umsatz wurde über eine Anpassung der Ofentemperatur justiert.

Die Vergleichstests wurden mit dem nach dem obigen Verfahren formierten Katalysator gemäß Beispiel 1 im Laborreaktor durchgeführt.

**Beispiel 4: Erfindungsgemäße Herstellung des MSA-Katalysators unter Einsatz von Aminen als Strukturmodifizierer:**

Die Herstellung des Katalysators erfolgte gemäß der im Beispiel 1 beschriebenen Methode. An Stelle von Benzylalkohol wurden jedoch 27,8 g 1-Aminododecan (Vanadium : Aminodecan 1 : 0,05) der Mischung aus iso-Butanol und Cyclohexan zugesetzt. Die Aufarbeitung erfolgte wie in Beispiel 1 beschrieben.

**Beispiel 5 : Erfindungsgemäße Herstellung des MSA-Katalysators unter Einsatz von Phosphorverbindungen als Strukturmodifizierer:**

Die Herstellung des Katalysators erfolgte gemäß der in Beispiel 1 beschriebenen Methode. Der Zusatz des Strukturmodifizierers erfolgte in diesem Fall zweckmäßigerweise über die Phosphorsäure-Mischung. Diesem Gemisch wurden 37,5 g Tributylphosphit (Vanadium : Tributylphosphit = 1 : 0,05) zugesetzt und wie im Beispiel 1 aufgeführt der Reaktionsmischung zugegeben. Die Aufarbeitung erfolgte wie in Beispiel 1 beschrieben.

**Beispiel 6: Erfindungsgemäße Herstellung von MSA-Katalysatoren unter Zusatz von Promotoren**

a) Die Herstellung des Katalysator erfolgte gemäß der in Beispiel 1 aufgeführten Methode. Als Promotor wurde in diesem Fall eine im verwendeten Lösungsmittel-Gemisch lösliche Kobaltverbindung (z.B. Kobaltacetylacetonat = $Co(acac)_2$) verwendet. Dem Lösungsmittel aus Beispiel 1 wurden 15,43 g $Co(acac)_2$ (acac = Pentan-2,4-dion, Acetylaceton), entsprechend einem Vanadium : Co-Verhältnis von 1 : 0,02, zugesetzt. Die restliche Reaktion und Aufarbeitung erfolgte wie in Beispiel 1 beschrieben.

b) In diesem Beispiel wurde eine Imprägnierung des gebildeten Katalysatorvorläufers mit einem Dotierstoff beschrieben. Nach Herstellung des Katalysatorvorläufers gemäß Beispiel 1 wurde der Reaktionsmischung vor der Abtrennung des Lösungsmittels durch Trocknung eine Lösung von 15,81 g $Zn(acac)_2$ in 100 ml iso-Butanol, entsprechend einem Vanadium : Zn-Verhältnis von 1 : 0,02, zugefügt. Nach Durchmischung erfolgte die Aufarbeitung des Reaktionsgemisch über eine Trocknungsmethode.

Die weitere Aufarbeitung und Calcinierung erfolgte wie in Beispiel 1 beschrieben.

Vergleichstest von MSA-Katalysatoren nach Verfahren 1 bis 3: Die zu untersuchenden Katalysatoren wurden gemäß den in den Verfahren 1 bis 3 beschriebenen Methode hergestellt (einschl. Formierung bzw. Calcinierung). Der Test selbst wurde wie im Beispiel 1 aufgeführt im Laborreaktor durchgeführt. Die Temperatur des Ofensystem wurde dabei jeweils solange variiert, bis eine maximale Ausbeute erreicht wurde. Die Ergebnisse der Tests (bei maximaler Ausbeute) sind in der folgende Tabelle aufgeführt:

Tabelle 2:

| Vergleich der Leistungsfähigkeit von MSA-Katalysatoren nach verschiedenen Herstellverfahren | | | |
|---|---|---|---|
| | Katalysator Nach Verfahren 1 (erfindungsgemäß) | Katalysator nach Verfahren 2 (Pantochim) | Katalysator nach Verfahren 3 (Hüls) |
| Ofentemperatur in °C | 365 | 355 | 375 |
| Umsatz in % | 93.1 | 93.5 | 87.8 |
| Ausbeute in mol-% | 59.2 | 57.8 | 52.7 |
| Selektivität in % | 63.6 | 61.8 | 60.0 |

Aus der obigen Tabelle zeigt sich deutlich die Überlegenheit der in dieser Schrift beschriebenen Herstellungsmethode gegenüber dem Verfahren 3. Die Überlegenheit gegenüber dem Verfahren 3 zeigt sich dabei zum einen in der um 6,5 mol-% höheren Ausbeute bei der um 10°C niedrigeren Ofentemperatur. Gleichzeitig zeigt der mit ca. 88 % relativ niedrige Umsatz bei maximaler Ausbeute auch eine geringe Aktivität der nach Verfahren 3 hergestellten Katalysators.

Gegenüber dem Verfahren 2 sind die Vorteile der erfindungsgemäßen Herstellungsverfahrens 1 weniger stark ausgeprägt. Es wird aber eine um 1,4 mol-% höhere Ausbeute des nach dem erfindungsgemäßen Verfahrens hergestellten Katalysator gefunden. Die Bedeutung dieses Ausbeuteunterschieds wird deutlich, wenn die gesamte Mehrproduktion durch einen solchen Katalysator mit den Kosten der Katalysatorfüllung verglichen wird. Ein Ausbeuteunterschied in der obigen Größenordnung führt dazu, daß die Kosten für die Katalysatorfüllung innerhalb der Katalysator-Lebenszeit durch die höhere Ausbeute erwirtschaftet werden.

Zusammenfassend zeigen diese Versuchsergebnisse die deutliche Überlegenheit des in dieser Schrift beschriebenen Herstellungsverfahrens. Zusätzlich ergeben sich durch den Verzicht auf hochkonzentrierte Polyphosphorsäure (gem. Verfahren 2) und den Einsatz von Phosphoriger Säure als Reduktionsmittel deutliche Handhabungsvorteile. Durch den Einsatz von Dotierelementen bzw. Strukturmodifizierern ('Templaten') kann die Aktivität der Katalysatoren optimal auf die Erfordernisse angepaßt werden.

**Patentansprüche**

**1.** Verfahren zur Herstellung von Katalysatoren für die Synthese von Maleinsäureanhydrid durch Oxidation von ge-

sättigten und/oder ungesättigten $C_4$-Kohlenwasserstofen auf der Basis von Vanadylphosphaten durch Umsetzung einer Vanadium(V)-Verbindung mit einer Mischung aus Phosphoriger- und Phosphorsäure mit einem molaren $H_3PO_3/H_3PO_4$-Verhältnis von 1:1 bis 1:2,5 in einem Iso-Butanol enthaltenden Lösungsmittelgemisch, **dadurch gekennzeichnet, daß** in dem Lösungsmittelgemisch ein Strukturbildner aus der Gruppe enthaltend, ein- und mehrfunktionelle Alkohole, ein- und mehrfunktionelle Amine, organische Phosphate, Phosphite und Phosphonate, ein Schleppmittel aus der Gruppe enthaltend, Alkylaromaten und Cycloalkane, wobei das Molverhältnis zwischen Vanadium und Strukturbildner in einem Bereich zwischen 10 : 1 und 100 : 1 liegt, eingesetzt wird und bei Siedetemperatur das Reaktionswasser mit Schleppmittel kontinuierlich ausgekreist wird, wobei der Restwassergehalt des Rücklaufs und der sich daraus ergebende Restwassergehalt in der Reaktionsmischung unter 0,5 Vol.-% liegt und der so erhaltene Precursor anschließend einer Calcinierung unterzogen wird, bei der ein Temperaturprogramm bestehend aus 3 Rampen zum Einsatz kommt, wobei die Rampen wie folgt gestaltet werden:

→ Rampe 1: Starttemperatur bei ≤ 50°C, Aufheizrate 5 bis 20°C/min, Endtemperatur 100 bis 250°C, Haltezeit 0 bis 3 h,

→ Rampe 2: Starttemperatur bei Endtemperatur Rampe 1, Aufheizrate 1 bis 10°C/min, Endtemperatur 150 bis 300°C, Haltezeit 0 bis 3 h,

→ Rampe 3: Starttemperatur bei Endtemperatur Rampe 2, Aufheizrate 0,1 bis 3°C/min, Endtemperatur: 380 bis 460°C, Haltezeit 2 bis 8 h,

und während dieser Temperaturrampen Gasgemische aus Luft, Inertgas und Wasserdampf in einem Volumen-Mischungsverhältnis zwischen 0,1 bis 0,5 : 0,1 bis 0,5 : 0,0 bis 0,8 eingesetzt werden.

2. Verfahren nach Anspruch 1, **dadurch gekennzeichnet, daß** das Atomzahlverhältnis Vanadium : Phosphor zwischen der eingesetzten Vanadiummenge und der Phosphormenge im Bereich von 1 : 0,9 bis 1 : 2,0 liegt, und die so erhaltene Verbindung eine mittlere Vanadiumoxidationsstufe von 3,85 bis 4,3 besitzt, wobei das molare Vanadium-Phosphor-Verhältnis in der isolierten Verbindung im Bereich zwischen 1 : 0,9 und 1 : 1,1 liegt.

3. Verfahren nach Anspruch 1 und 2, **dadurch gekennzeichnet, daß** als Strukturmodifizierer eine oder mehrere Verbindungen aus der Gruppe umfassend Ethandiol, Propan-1,3-diol, n-Hexanol, Diphenylmethanol, Benzylalkohol, alpha- omega-Alkandiole, n-Propylamin, iso-Butylamin, Ethylendiamin, n-Decylamin, Propylendiamin, Butylphosphat, Dibutylphosphat, Trimethylphosphat, Tributylphosphat, Methylphosphonsäure, Trimethylphosphit, Dibutylphosphonsäure und Tributylphosphit, eingesetzt werden.

4. Verfahren nach Anspruch 1 bis 3, **dadurch gekennzeichnet, daß** als Schleppmittel eine oder mehrere Verbindungen aus der Gruppe umfassend Toluol, Xylole, Cyclohexan und Benzol zugesetzt wird.

5. Verfahren nach Anspruch 1 bis 4, **dadurch gekennzeichnet, daß** der Reaktionsmischung einer oder mehrere Dotierstoffe in Form löslicher Verbindungen der Elemente Mo, Wo, Bi, Cr, Co, Ni, Fe, Li, Ce, Zr, U und Zn in einem Atomzahlverhältnis Vanadium : Dotierelement von 1 : 0,1 bis 0,001 zugesetzt werden.

6. Verfahren nach Anspruch 1 bis 5, **dadurch gekennzeichnet, daß** die erhaltene Verbindung nach Trocknung mit einem oder mehreren Hilfsstoffen, bevorzugt Graphit, versehen wird und zu geeigneten Formkörpern durch Tablettierung, Agglomerierung, Extrusion geformt wird.

7. Verfahren nach Anspruch 1 bis 6, **dadurch gekennzeichnet, daß** die erhaltenen Formkörper mit Hilfe eines geeigneten Calcinierverfahrens extern oder in-situ unter definierter Gaskonzentration und Temperaturbedingung in die aktive Verbindung überführt werden.

8. Verfahren nach Anspruch 1 bis 7, **dadurch gekennzeichnet, daß** vor Beginn des Temperaturprogramms eine Inertisierung des Calciniersystems mit Inertgas erfolgt, so daß ein Sauerstoffgehalt von unter 5 Vol.-% erreicht wird.

9. Verfahren nach Anspruch 1 bis 8, **dadurch gekennzeichnet, daß** nach Abschluß des Temperaturprogramms die Abkühlung des Calciniersystems auf eine Temperatur von kleiner 100°C unter Inertgasatmosphäre erfolgt.

10. Katalysator auf der Basis von Vanadylphosphaten für die Gasphasenoxidation von C4-Kohlenwasserstoffen zu Maleinsäureandydrid gemäß eines Verfahrens nach Anspruch 1 bis 9, **dadurch gekennzeichnet, daß** das Atomzahlverhältnis Vanadium : Phosphor zwischen der eingesetzten Vanadiummenge und der Phosphormenge im Bereich von 1 : 0,9 bis 1 : 2,0 liegt, und die so erhaltene Verbindung eine mittlere Vanadiumoxidationsstufe von 3,85 bis 4,3 besitzt, wobei das molare Vanadium-Phosphor-Verhältnis in der isolierten Verbindung im Bereich zwischen 1 : 0,9 und 1 : 1,1 liegt.

**Claims**

1.  Process for preparing catalysts based on vanadyl phosphates for the synthesis of maleic anhydride by oxidation of saturated and/or unsaturated $C_4$-hydrocarbons by reacting a vanadium(V) compound with a mixture of phosphorous and phosphoric acids with a molar $H_3PO_3/H_3PO_4$ ratio of from 1 : 1 to 1 : 2.5 in a solvent mixture containing isobutanol, **characterized in that** a structure former selected from the group consisting of monofunctional and polyfunctional alcohols, monofunctional and polyfunctional amines, organic phosphates, phosphites and phosphonates, and an entrainer selected from the group consisting of alkylaromatics and cycloalkanes, where the molar ratio of vanadium to structure former is in the range from 10 : 1 to 100 : 1, are used in the solvent mixture, and the water of reaction together with entrainer is continuously removed at the boiling point, where the residual water content of the runback and the resulting residual water content in the reaction mixture are less than 0.5% by volume, and the precursor obtained in this way is subsequently subjected to a calcination, in which a temperature programme comprising 3 ramps is used, where the ramps are as follows:

    →  ramp 1: initial temperature at ≤ 50°C, heating rate = 5 to 20°C/min, final temperature = 100 to 250°C, hold time = 0 to 3 h,
    →  ramp 2: initial temperature at final temperature of ramp 1, heating rate = 1 to 10°C/min, final temperature = 150 to 300°C, hold time = 0 to 3 h,
    →  ramp 3: initial temperature at final temperature of ramp 2, heating rate = 0.1 to 3°C/min, final temperature = 380 to 460°C, hold time = 2 to 8 h,

    and gas mixtures of air, inert gas and water vapour in a volume mixing ratio of 0.1-0.5 : 0.1-0.5 : 0.0-0.8 are used during the temperature ramps.

2.  Process according to Claim 1, **characterized in that** the vanadium compound and the phosphorus compounds are used in such amounts that the atom number ratio of vanadium:phosphorus is in the range from 1 : 0.9 to 1 : 2.0 and the vanadium-phosphorus compound obtained in this way has a mean vanadium oxidation state of from 3.85 to 4.3, with the molar vanadium:phosphorus ratio in the isolated compound being in the range from 1 : 0.9 to 1 : 1.1.

3.  Process according to Claim 1 or 2, **characterized in that** the structure modifier used comprises one or more compounds selected from the group consisting of ethanediol, 1,3-propanediol, n-hexanol, diphenylmethanol, benzyl alcohol, alpha-omega-alkanediols, n-propylamine, isobutylamine, ethylenediamine, n-decylamine, propylenediamine, butyl phosphate, dibutyl phosphate, trimethyl phosphate, tributyl phosphate, methylphosphonic acid, trimethyl phosphite, dibutylphosphonic acid, and tributyl phosphite.

4.  Process according to any of Claims 1 to 3, **characterized in that** the entrainer added comprises one or more compounds selected from the group consisting of toluene, xylenes, cyclohexane and benzene.

5.  Process according to any of Claims 1 to 4, **characterized in that** one or more dopants in the form of soluble compounds of the elements Mo, Wo, Bi, Cr, Co, Ni, Fe, Li, Ce, Zr, U and Zn are added to the reaction mixture in an atom number ratio of vanadium:doping element of from 1 : 0.1 to 1 : 0.001.

6.  Process according to any of Claims 1 to 5, **characterized in that** the compound obtained is, after drying, provided with one or more auxiliaries, preferably graphite, and shaped to form suitable shaped bodies by tableting, agglomeration, extrusion.

7.  Process according to any of Claims 1 to 6, **characterized in that** the shaped bodies obtained are converted into the active compound by means of a suitable calcination process either externally or in situ under a defined gas concentration and temperature conditions.

8.  Process according to any of Claims 1 to 7, **characterized in that** the calcination system is made inert using inert gas prior to commencement of the temperature programme, so that an oxygen content of less than 5% by volume is achieved.

9. Process according to any of Claims 1 to 8, **characterized in that**, after the temperature programme has ended, the calcination system is cooled under inert gas to a temperature of less than 100°C.

10. Catalyst based on vanadyl phosphates for the gas-phase oxidation of $C_4$-hydrocarbons to form maleic anhydride by a process according to any of Claims 1 to 9, **characterized in that** the vanadium compound and the phosphorus compounds are used in such amounts that the atom number ratio of vanadium:phosphorus is in the range from 1 : 0.9 to 1 : 2.0 and the vanadium-phosphorus compound obtained in this way has a mean vanadium oxidation state of from 3.85 to 4.3, with the molar vanadium:phosphorus ratio in the isolated compound being in the range from 1 : 0.9 to 1 : 1.1.

**Revendications**

1. Procédé pour la préparation de catalyseurs pour la synthèse de l'anhydride de l'acide maléique par oxydation d'hydrocarbures en $C_4$ saturés et/ou insaturés à base de phosphates de vanadyle par transformation d'un composé du vanadium (V) avec un mélange d'acide phosphoreux et d'acide phosphorique avec un rapport molaire $H_3PO_3$/ $H_3PO_4$ de 1:1 à 1:2,5 dans un mélange de solvants contenant de l'isobutanol, **caractérisé en ce qu'**on utilise dans le mélange de solvants un agent de formation de structure du groupe contenant des alcools monovalents et polyvalents, des amines monovalentes et polyvalentes, des phosphates, phosphites et phosphonates organiques, un agent d'entraînement du groupe contenant des aromatiques d'alkyle et des cycloalcanes, le rapport molaire entre le vanadium et l'agent de formation de structure étant compris dans une plage entre 10:1 et 100:1 et qu'à la température d'ébullition, l'eau de réaction est enlevée en continu du circuit avec l'agent d'entraînement, la teneur en eau résiduelle du retour et la teneur en eau résiduelle qui en résulte dans le mélange réactionnel étant inférieure à 0,5% en volume et le précurseur ainsi obtenu étant ensuite soumis à une calcination dans laquelle on utilise un programme de température constitué de trois rampes, les rampes étant constituées comme suit :

   → Rampe 1 = température de démarrage ≤ 50°C, vitesse de chauffage 5 à 20°C/min, température finale 100 à 250°C, durée de maintien 0 à 3 heures,
   → Rampe 2 : température de démarrage à la température finale de la rampe 1, vitesse de chauffage 1 à 10°C/min, température finale 150 à 300°C, durée de maintien 0 à 3 heures,
   → Rampe 3 : température de démarrage à la température finale de la rampe 2, vitesse de chauffage 0,1 à 3°C/min, température finale 380 à 460°C, durée de maintien 2 à 8 heures,

   et des mélanges gazeux d'air, de gaz inerte et de vapeur d'eau étant utilisés dans un rapport de mélange volumique entre 0,1 à 0,5 : 0,1 à 0,5 : 0,0 à 0,8 pendant ces rampes de température.

2. Procédé selon la revendication 1, **caractérisé en ce que** le rapport de nombres atomiques vanadium : phosphore entre la quantité utilisée de vanadium et la quantité de phosphore est situé dans la plage de 1 : 0,9 à 1 : 2,0 et que le composé ainsi obtenu présente un degré d'oxydation moyen du vanadium de 3,85 à 4,3, le rapport molaire vanadium-phosphore dans le composé isolé étant situé dans la plage entre 1 : 0,9 et 1 : 1,1.

3. Procédé selon les revendications 1 et 2, **caractérisé en ce qu'**on utilise comme modificateur de structure un ou plusieurs composés du groupe constitué d'éthanediol, de propane-1,3-diol, de n-hexanol, de diphénylméthanol, d'alcool benzylique, d'alpha-oméga-alcanediols, de n-propylamine, d'isobutylamine, d'éthylènediamine, de n-décylamine, de propylènediamine, de phosphate de butyle, de phosphate de dibutyle, de phosphate de triméthyle, de phosphate de tributyle, d'acide méthylphosphonique, de phosphite de triméthyle, d'acide dibutylphosphonique et de phosphite de tributyle.

4. Procédé selon les revendications 1 à 3, **caractérisé en ce qu'**on ajoute comme agent d'entraînement un ou plusieurs composés du groupe constitué du toluène, des xylènes, du cyclohexane et du benzène.

5. Procédé selon les revendications 1 à 4, **caractérisé en ce qu'**on ajoute au mélange réactionnel un ou plusieurs dopants sous forme de composés solubles des éléments Mo, Wo, Bi, Cr, Co, Ni, Fe, Li, Ce, Zr, U et Zn dans un rapport de nombres atomiques vanadium : élément dopant de 1 : 0,1 à 0,001.

6. Procédé selon les revendications 1 à 5, **caractérisé en ce que** le composé obtenu est pourvu, après séchage, d'un ou de plusieurs adjuvants, de préférence du graphite, et est façonné en corps façonnés appropriés par formation de comprimés, par agglomération, par extrusion.

7. Procédé selon les revendications 1 à 6, **caractérisé en ce que** les corps façonnés obtenus sont transformés en composé actif à l'aide d'un procédé de calcination approprié externe ou in situ, sous une concentration en gaz définie et une condition de température.

8. Procédé selon les revendications 1 à 7, **caractérisé en ce qu'**avant le début du programme de température, on rend le système de calcination inerte avec du gaz inerte, de manière à atteindre une teneur en oxygène inférieure à 5% en volume.

9. Procédé selon les revendications 1 à 8, **caractérisé en ce qu'**après le programme de température, le refroidissement du système de calcination est réalisé à une température inférieure à 100°C sous une atmosphère de gaz inerte.

10. Catalyseur à base de phosphates de vanadyle pour l'oxydation en phase gazeuse d'hydrocarbures en $C_4$ en anhydride de l'acide maléique selon un procédé selon les revendications 1 à 9, **caractérisé en ce que** le rapport de nombres atomiques vanadium : phosphore entre la quantité utilisée de vanadium et la quantité de phosphore est situé dans la plage de 1 : 0,9 à 1 : 2,0 et que le composé ainsi obtenu présente un degré d'oxydation moyen du vanadium de 3,85 à 4,3, le rapport molaire vanadium-phosphore dans le composé isolé étant situé dans la plage entre 1 : 0,9 et 1 : 1,1.